# EUROPEAN PATENT SPECIFICATION

(11) **EP 0 663 208 B1**
(45) Date of publication and mention of the grant of the patent: **02.05.1997**
(21) Application number: 94650039.4
(22) Date of filing: 19.12.1994
(51) Int. Cl.: A61K 9/00, A61K 33/10

(54) **Combinations of polymers for use in artificial tear compositions**
Kombinierten Polymere zur Verwendung in künstlicher Tränenflussigkeit
Combinaisons de polymères pour utilisation dans des larmes artificielles

(30) Priority: 20.12.1993 US 170482
(43) Date of publication of application: 19.07.1995
(73) Proprietor: ALCON LABORATORIES, INC., Fort Worth Texas 76107 (US)
(72) Inventor: Bhagat, Haresh G., Fort Worth, Texas 76133 (US)
(74) Representative: Hanna, Peter William Derek

(56) References cited:
- EP-A- 0 205 279
- EP-A- 0 546 728
- DATABASE WPI Week 8728 Derwent Publications Ltd., London, GB; AN 87-194483 & JP,A,62 122 671 (SENJU SEIYAKU KK)

## Description

### Background of the Invention

This invention relates generally to ophthalmic compositions. In particular, the present invention relates to artificial tear compositions comprising the ionic components of normal human tear film in substantially the same amounts and proportions, as well as to methods for their preparation and storage. These compositions are useful as lubricating and cushioning agents for the eye after traumatic injury or surgery. The present invention also relates to a method of treating eyes by topically applying the compositions of the present invention when indicated for the relief of dry eye syndrome and when indicated to achieve the other effects mentioned above.

Dry eye syndrome and related ailments, including transitory discomforts, are well known in the scientific and patent literature. These ailments have generally been treated by topical administration of any of a number of ophthalmic compositions. The currently marketed artificial tear compositions are listed on pages 504-504b of Drug Facts and Comparisons New York: J.B. Lippincott Co., 1989. In general, these compositions contain salts, buffers and viscosity agents (e.g., hydroxypropyl methylcellulose, polyvinyl alcohol or Carbopol®, a carboxy vinyl polymer). Most artificial tear compositions additionally contain preservatives (e.g., benzalkonium chloride, Dymed®, a biguanide, and Polyquad®, a polymeric quaternary ammonium compound), although some recently introduced compositions are non-preserved.

It has recently been determined that preservatives and non-physiologic ions which may be present in artificial tear compositions may be detrimental to the corneal epithelium. See, for example, Bernal et al., Current Eye Research, 10(7):645-656 (1991). There have therefore been attempts to develop non-preserved artificial tear compositions containing physiological tear components. See, for example, U.S. Patent No. 4,775,531 (Gilbard); however, these formulations are based on the composition of rabbit tears and it has now been documented that human tears, although having the same types of ions, have distinctly different ion concentrations. See Rismondo et al., in The Contact Lens Association of Ophthalmologists, 15(3):222-229 (1989). In addition, although Gilbard's compositions list bicarbonate as an ingredient, bicarbonate is quite labile because it is in equilibrium with carbon dioxide, and can escape from solution in a relatively short time.

EP-A-0,546,728 (Alcon Laboratories, Inc.) discloses non-preserved physiological tear compositions comprising ionic components of normal human tears in substantially the same amounts and proportions with hydroxypropyl methylcellulose as the sole polymer in the composition.

JP-A-62122671 (Senju Seiyaku K.K.) discloses an opthalmic solution for preventing corneal damage in eye surgery, comprising hydroxypropyl methylcellulose and/or hyaluronate at preferably 1.0 to 2.0% w/w in a buffered solution containing salts and/or sugars adjusted to a pH of 6 to 8, with a viscosity of 1000-10000 cps.

### Summary of the Invention

The compositions of the present invention are non-preserved compositions which contain the essential ionic components of normal human tear film in substantially the same amounts and proportions and which avoid some of the problems of known compositions. In addition, it has surprisingly been found that compositions containing bicarbonate are substantially more effective in treating dry eye syndrome and its related ailments, than currently available artificial tear preparations.

Additionally, the present invention is based on the finding that compositions comprising certain combinations of at least one cellulosic polymer and at least one carbon vinyl polymer or a glycosaminoglycan are much more viscous than similar compositions containing only one type of polymer. That is, a lower polymer concentration is required to achieve a higher viscosity when polymer combinations of the present invention are used than when only one of the polymers is used. This is particularly beneficial in the ophthalmic field, because a reduction in the overall polymer concentration in an ophthalmic composition generally results in greater patient comfort. Using the polymer combinations of the present invention, higher viscosity can be achieved without increasing the overall polymer concentration. This high viscosity aids in the retention of the composition in the eye and permits the maintenance of the moisturizing effect, of particular importance with respect to artificial tear formulations.

Further, the compositions of the present invention are prepared by an unique method which involves the use of CO₂ gas in order to retain bicarbonate in solution during preparation. The amount of bicarbonate dissolved in the solution depends on the components and conditions in the solution, as well as the conditions of the atmosphere surrounding the solution. An equilibrium is established which depends on these parameters, as described in the equation below:

PCO₂<<<< >>>> Dissolved CO₂<<<< >>>> H₂CO₃<<< >>> H⁺+ HCO₃⁻

(where PCO₂ is the partial pressure of CO₂ above the solution). The bicarbonate concentration is maintained during storage by use of the novel packaging of the present invention, which creates a closed system in which an equilibrium between CO₂ and bicarbonate can be reached and maintained until the composition is to be used.

### Detailed Description of the Invention

In general, the physiological tear compositions of the present invention comprise the following ion components: potassium at a concentration of between about 11 and about 25 millimoles per liter (mmol/L); calcium at a concentration of between about 0.2 and about 0.5 mmol/L; magnesium at a concentration of between about 0.15 and about 0.45 mmol/L; and bicarbonate at a concentration of between about 1 and about 36 mmol/L, preferably between about 6 and about 24 mmol/L. The compositions may additionally contain zinc at a concentration between about 0.005 and about 0.015 mmol/L. In a preferred composition, the potassium ion concentration is about 17.4 mmol/L, the calcium ion concentration is about 0.36 mmol/L, the magnesium ion concentration is about 0.31 mmol/L and the bicarbonate concentration is about 11.9 mmol/L. As used throughout this application, all concentrations refer to final composition concentrations, unless otherwise stated.

These physiological tear compositions preferably have certain ion ratios. In particular, it is preferred that: the molar concentration ratio of potassium to bicarbonate is between about 1:0.04 and about 1:3.27; the molar concentration ratio of calcium to magnesium is between about 1:0.3 and about 1:2.25; the molar concentration ratio of potassium to calcium is between about 1:0.008 and about 1:0.045; and the molar concentration ratio of bicarbonate to calcium is between about 1:0.0056 and about 1:0.5. Especially preferred are molar concentration ratios of: potassium to bicarbonate between about 1:0.24 and about 1:2.18; and bicarbonate to calcium between about 1:0.008 and about 1:0.08. Most preferred are the compositions wherein the molar concentration ratio of potassium to bicarbonate is about 1:0.68, the molar concentration ratio calcium to magnesium is about 1:0.86, the molar concentration ratio of potassium to calcium is about 1:0.02 and the molar concentration ratio of bicarbonate to calcium is about 1:0.03.

The compositions of the present invention further comprise a combination of cellulosic polymers and, glycosaminoglycans, or cellulosic polymers and carbon vinyl polymers. These viscous compositions will generally have a viscosity between about 5 and 15,000 centipoise (cps), preferably between about 2,000 and 10,000 cps, and most preferably between about 3,000 and about 6,000 cps. The viscosity of such compositions will vary, depending on the particular polymer combinations used and the relative concentrations of each polymer.

The cellulosic polymers useful in the viscous compositions of the present invention include all cellulose derivatives which exhibit viscoelastic properties. In general, such cellulosic polymers have an average molecular weight between about 10,000 and 13 million Preferred cellulosic polymers include: hydroxypropyl methyl cellulose (HPMC), hydroxyethyl cellulose (HEC), hydroxypropyl cellulose (HPC) and methyl cellulose (MC). In general, these cellulosic polymers are present in the compositions of the present invention at a concentration between about 0.05 and about 5.0 percent by weight (wt%), preferably between about 0.25 and about 1.0 wt%, and most preferably at about 0.5% wt%.

The glycosaminoglycans (GAGs) useful in the viscous compositions of the present invention include chondroitin sulfate (COS), hyaluronic acid (HA), and keratan sulfate. These GAGs have average molecular weights between about 25,000 and about 2,000,000. In general, the GAGs are present in the compositions of the present invention at a concentration between about 0.1 and about 10.0 wt%, preferably between about 0.2 and about 5.0 wt%.

The carboxy vinyl polymers useful in the viscous compositions of the present invention have an average molecular weight between about 500,000 and 6 million. The polymers are characterized as having carboxylic acid functional groups and preferably contain between 2 and 7 carbon atoms per functional group. Suitable carboxy vinyl polymers include those called carbomers, e.g., Carbopol® (B.F. Goodrich Co., Cleveland, Ohio). Specifically preferred are: carbomer 910, carbomer 940, carbomer 934P, carbomer 974P, carbomer 980 and carbomer 1342. Carbomer 934P and carbomer 974P are the most preferred. Such polymers will typically be employed in an amount between about 0.05 and about 3.0 wt%, depending on the desired viscosity of the composition. Preferably, the carboxy vinyl polymer is present at a concentration between abut 0.1 and about 0.5 wt%, and most preferably at a concentration of about 0.175 wt%.

The preferred compositions of the present invention are viscous, physiological tear compositions which include the ion components and the viscosity enhancing polymer combinations detailed above.

The compositions of the present invention may contain one or more pharmaceutically active agents ("actives"). Such actives include, but are not limited to: glaucoma agents, such as miotics (e.g., pilocarpine, carbachol and acetylcholinesterase inhibitors), sympathomimetics (e.g., epinephrine, dipivalylepinephrine and para-amino clonidine), beta-blockers (e.g., betaxolol, levobunolol and timolol) and carbonic anhydrase inhibitors (e.g., acetazolamide, methazolamide and ethoxzolamide); dopaminergic antagonists; antihypertensive agents, such as para-amino clonidine (also known as apraclonidine); anti-infectives, such as ciprofloxacin; non-steroidal and steroidal anti-inflammatories, such as suprofen, ketorolac, tetrahydrocortisol, dexamethasone, diclofenac and rimexolone; prostaglandins; retinoids; aldose reductase inhibitors; proteins; growth factors, such as epidermal growth factor; and anti-allergics.

The compositions of the present invention may contain sodium chloride at a concentration between about 75 and about 154 mmol/L so that the osmolality is between about 200 and about 350 millimoles/kilogram (mOsm/kg). It is preferred that the compositions have an osmolality of between about 260 and about 330 mOsm/kg. The compositions of the present invention will have a pH between about 5.0 and about 9.5. It is preferred that the compositions have a pH between about 5.5 and 8.5.

The compositions of the present invention additionally may contain mucomimetic polymers and lubricating agents for increased comfort and sustained duration in the eye. Examples of the above include: Dextran; polyvinyl pyrrolidone; and polyethylene glycols. In general, these polymers are present in the compositions of the present invention at a concentration between about 0.05 and about 5.0 percent by weight (wt%), preferably between about 0.1 and about 2.0 wt%.

The compositions of the present invention may be prepared by dissolving or dispersing all of the ingredients in purified water in a pressure vessel. The components are mixed and the reactor heated to a suitable temperature for a time sufficient to achieve assured sterilization, according to common sterilization procedures. The mixture is then cooled to room temperature with mixing. In the alternative, a solution of bicarbonate which has previously been sterilized by filtration may be added at this stage. The pH of the composition is adjusted to the desired range (between 5.6 and 7.9) by use of sterile carbon dioxide and mixing the contents of the reactor. Sodium hydroxide and/or hydrochloric acid may additionally be used to adjust the pH of the mixture. The final product is then aseptically filled according to procedures known in the art. In another alternative, all of the ingredients are dissolved or dispersed in purified water, followed by pH adjustment as described above. Sterilization may be accomplished either by filtration of the composition into a pressure vessel prior to the pH adjustment or by filtration of the composition directly into the filling machine after pH adjustment.

The packaging for the compositions of the present invention preferably are made from a material that is relatively impermeable with respect to the gas contained in the composition. For example, if the gas is carbon dioxide, laminated foil or some high density plastics are suitable packaging materials. The final packaging may consist of multiple layers of packaging. The choice of material will in part depend on the desired product shelf life; i.e., the longer the desired shelf life, the more impermeable the material needs to be. The compositions of the present invention containing bicarbonate are preferably packaged in unit dose containers which are then sealed into laminated foil pouches. The manufacture and filling of such unit dose containers are known in the art (generally referred to as "form, fill and seal"). Multiple unit dose containers may be packaged in each laminated foil pouch.

Although it is preferred that the compositions of the present invention containing bicarbonate be packaged in unit dose containers, it is understood that multidose non-preserved dispensing package systems could be used, so long as the packaging contains an appropriate barrier to prevent or reduce the escape of gas. For example, a packaging system consisting of laminate tubes using dispensing tip assemblies such as those disclosed in US 4,917,271 (Kanner, et al.) and US 5,025,957 (Ranalletta, et al.), is suitable for the compositions of the present invention.

Sample formulations of the compositions of the present invention are listed in Table 1 below:

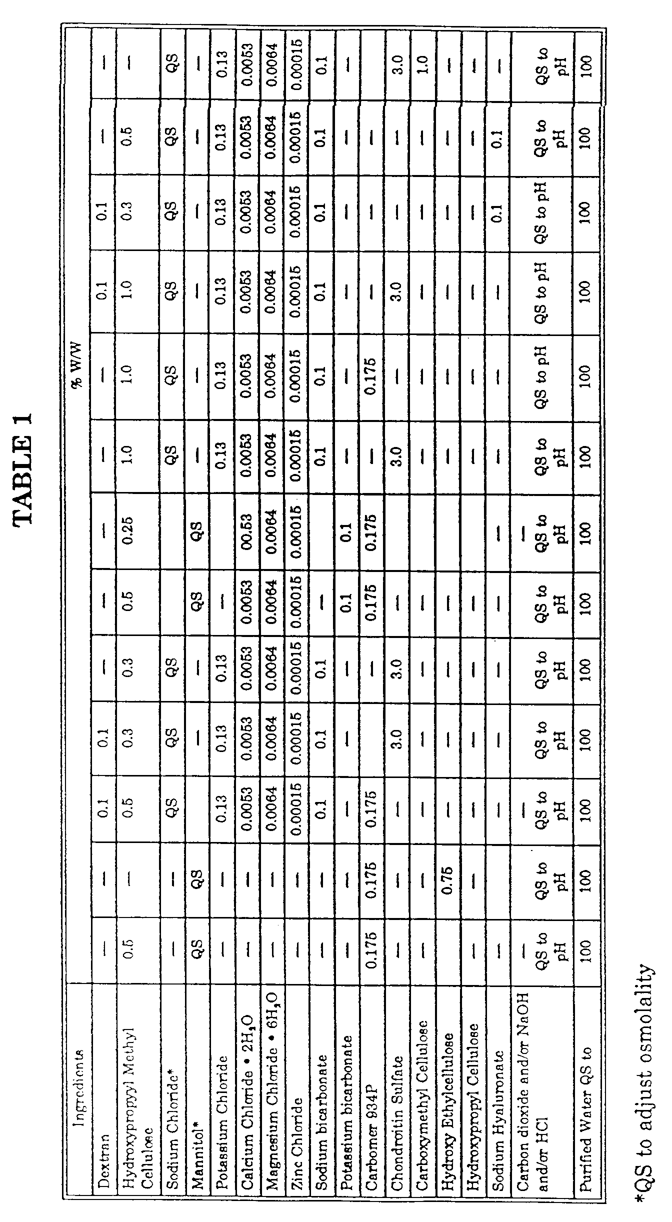

## Claims

1. An ophthalmic composition comprising:
a) potassium ions at a concentration between about 11 and about 25 mmol/L;
b) calcium ions at a concentration between about 0.2 and about 0.5 mmol/L;
c) magnesium ions at a concentration between about 0.15 and about 0.45 mmol/L;
d) bicarbonate ions at a concentration between about 1 and about 36 mmol/L;
e) the combination of:
a cellulosic polymer and a glycosaminoglycan, or a cellulosic polymer and a carboxy vinyl polymer; and
f) a viscosity between about 5 and about 10,000 centipoise.

2. The composition of claim 1, wherein the bicarbonate ion concentration is between 6 and 24 mmol/L.

3. The composition of claim 1, further comprising zinc ions at a concentration between about 0.005 and about 0.015 mmol/L.

4. The composition of claim 1, wherein the potassium ion concentration is about 17.4 mmol/L, the calcium ion concentration is about 0.36 mmol/L, the magnesium ion concentration is about 0.31 mmol/L and the bicarbonate ion concentration is about 11.9 mmol/L.

5. The composition of claim 1, wherein:
a) the molar concentration ratio of potassium to bicarbonate is between about 1:0.04 and about 1:3.27;
b) the molar concentration ratio of calcium to magnesium is between about 1:0.3 and about 1:2.25;
c) the molar concentration ratio of potassium to calcium is between about 1:0.008 and about 1:0.045; and
d) the molar concentration ratio of bicarbonate to calcium is between about 1:0.0056 and about 1:0.5.

6. The composition of claim 5, wherein the molar concentration ratio of potassium to bicarbonate is about 1:0.68, the molar concentration ratio of calcium to magnesium is about 1:0.86, the molar concentration ratio of potassium to calcium is about 1:0.02 and the molar concentration ratio of bicarbonate to calcium is about 1:0.03.

7. The composition of claim 5, wherein:
a) the molar concentration ratio of potassium to bicarbonate is between about 1:0.24 and about 1:2.18; and
b) the molar concentration ratio of bicarbonate to calcium is between about 1:0.008 and about 1:0.08

8. The composition of claim 1, wherein the composition has a viscosity between about 3,000 and about 6,000 centipoise.

9. The composition of claim 1, wherein the cellulosic polymer is hydroxypropyl methyl cellulose, hydroxyethyl cellulose, hydroxy propyl cellulose or methyl cellulose.

10. The composition of claim 1, wherein the carboxy vinyl polymer is carbomer 910, carbomer 940, carbomer 934P, carbomer 974P, carbomer 980 or carbomer 1342.

11. The composition of claim 1, wherein the cellulosic polymer comprises hydroxypropyl methyl cellulose and the carboxy vinyl polymer comprises carbomer 934P.

12. The composition of claim 1, wherein the cellulosic polymer is present at a concentration of about 0.5 percent by weight.

13. The composition of claim 1, wherein the carboxy vinyl polymer is present at a concentration of about 0.175 percent by weight.

14. The composition of claim 1, wherein the glycosaminoglycan is present in the composition at a concentration between about 0.1 and 5 percent by weight.

15. A process for preparing an ophthalmic composition according to any of claims 1 to 14 wherein the composition is prepared by the following steps:
i) mixing the composition ingredients in a suitable vessel;
ii) placing the composition in a pressure reactor vessel;
iii) charging the pressure reactor vessel with a quantity of a gas, wherein the quantity of the gas is sufficient to induce a desired equilibrium state between the gas and the bicarbonate within the closed system of the pressure reactor vessel; and
iv) mixing the contents of the pressure reactor vessel for a period of time sufficient to induce the equilibrium state between the gas and the bicarbonate.

16. The process of claim 15 wherein the gas is carbon dioxide.

17. The process of claim 15, wherein the suitable vessel for mixing the composition ingredients is the pressure reactor vessel.

18. The process of claim 15, further comprising transferring the contents of the pressure reactor vessel into a container without significant loss of the bicarbonate.

19. The process of claim 18, wherein the container is substantially impermeable to the gas contained therein.

20. The process of claim 19, wherein the container comprises a laminated foil pouch.

21. A package containing the ophthalmic composition according to any of claims 1 to 14, substantially impermeable to carbon dioxide contained above and dissolved in the composition, such that a closed system is created in which an equilibrium between carbon dioxide and bicarbonate can be reached and maintained until the package is opened for use.

## Patentansprüche

1. Eine ophthalmische Zusammensetzung, welche umfaßt:
a) Kalium-Ionen in einer Konzentration zwischen etwa 11 und etwa 25 mmol/l;
b) Calcium-Ionen in einer Konzentration zwischen etwa 0,2 und etwa 0,5 mmol/l;
c) Magnesium-Ionen in einer Konzentration zwischen etwa 0,15 und etwa 0,45 mmol/l;
d) Bicarbonat-Ionen in einer Konzentration zwischen etwa 1 und etwa 36 mmol/l;
e) die Kombination aus:
einem cellulosischen Polymer und einem Glycosaminoglycan oder einem cellulosischen Polymer und einem Carboxyvinylpolymer; und
f) eine Viskosität zwischen etwa 5 und etwa 10.000 Centipoise.

2. Die Zusammensetzung nach Anspruch 1, wobei die Bicarbonat-Ionen-Konzentration zwischen 6 und 24 mmol/l liegt.

3. Die Zusammensetzung nach Anspruch 1, die außerdem Zink-Ionen in einer Konzentration zwischen etwa 0,005 und etwa 0,015 mmol/l umfaßt.

4. Die Zusammensetzung nach Anspruch 1, wobei die Kalium-Ionen-Konzentration etwa 17,4 mmol/l beträgt, die Calcium-Ionen-Konzentration etwa 0,36 mmol/l beträgt, die Magnesium-Ionen-Konzentration etwa 0,31 mmol/l beträgt und die Bicarbonat-Ionen-Konzentration etwa 11,9 mmol/l beträgt.

5. Die Zusammensetzung nach Anspruch 1, wobei:
a) das molare Konzentrationsverhältnis von Kalium zu Bicarbonat zwischen etwa 1:0,04 und etwa 1:3,27 liegt;
b) das molare Konzentrationsverhältnis von Calcium zu Magnesium zwischen etwa 1:0,3 und etwa 1:2,25 liegt;
c) das molare Konzentrationsverhältnis von Kalium zu Calcium zwischen etwa 1:0,008 und etwa 1:0,045 liegt; und
d) das molare Konzentrationsverhältnis von Bicarbonat zu Calcium zwischen etwa 1:0,0056 und etwa 1:0,5 liegt.

6. Die Zusammensetzung nach Anspruch 5, wobei das molare Konzentrationsverhältnis von Kalium zu Bicarbonat etwa 1:0,68 beträgt, das molare Konzentrationsverhältnis von Calcium zu Magnesium etwa 1:0,86 beträgt, das molare Konzentrationsverhältnis von Kalium zu Calcium etwa 1:0,02 beträgt und das molare Konzentrationsverhältnis von Bicarbonat zu Calcium etwa 1:0,03 beträgt.

7. Die Zusammensetzung nach Anspruch 5, wobei:
a) das molare Konzentrationsverhältnis von Kalium zu Bicarbonat zwischen etwa 1:0,24 und etwa 1:2,18 liegt; und
b) das molare Konzentrationsverhältnis von Bicarbonat zu Calcium zwischen etwa 1:0,008 und etwa 1:0,08 liegt.

8. Die Zusammensetzung nach Anspruch 1, wobei die Zusammensetzung eine Viskosität zwischen etwa 3.000 und etwa 6.000 Centipoise besitzt.

9. Die Zusammensetzung nach Anspruch 1, wobei das cellulosische Polymer Hydroxypropylmethylcellulose, Hydroxyethylcellulose, Hydroxypropylcellulose oder Methylcellulose ist.

10. Die Zusammensetzung nach Anspruch 1, wobei das Carboxyvinylpolymer Carbomer 910, Carbomer 940, Carbomer 934P, Carbomer 974P, Carbomer 980 oder Carbomer 1342 ist.

11. Die Zusammensetzung nach Anspruch 1, wobei das cellulosische Polymer Hydroxypropylmethylcellulose umfaßt und das Carboxyvinylpolymer Carbomer 934P umfaßt.

12. Die Zusammensetzung nach Anspruch 1, wobei das cellulosische Polymer in einer Konzentration von etwa 0,5 Gewichtsprozent vorhanden ist.

13. Die Zusammensetzung nach Anspruch 1, wobei das Carboxyvinylpolymer in einer Konzentration von etwa 0,175 Gewichtsprozent vorhanden ist.

14. Die Zusammensetzung nach Anspruch 1, wobei das Glycosaminoglycan in der Zusammensetzung in einer Konzentration zwischen etwa 0,1 und 5 Gewichtsprozent vorhanden ist.

15. Ein Verfahren zur Herstellung einer ophthalmischen Zusammensetzung nach einem der Ansprüche 1 bis 14, wobei die Zusammensetzung mit den folgenden Schritten hergestellt wird:
i) Mischen der Inhaltsstoffe der Zusammensetzung in einem geeigneten Gefäß;
ii) Einbringen der Zusammensetzung in einen Druckreaktorgefäß;
iii) Beschicken des Druckreaktorgefäßes mit einer Menge eines Gases, wobei die Menge des Gases ausreichend ist, um einen gewünschten Gleichgewichtszustand zwischen dem Gas und dem Bicarbonat innerhalb des geschlossenen Systems des Druckreaktorgefäßes zu induzieren; und
iv) Mischen des Inhaltes des Druckreaktorgefäßes für einen Zeitraum, der ausreichend ist, um den Gleichgewichtszustand zwischen dem Gas und dem Bicarbonat zu induzieren.

16. Das Verfahren nach Anspruch 15, wobei das Gas Kohlendioxid ist.

17. Das Verfahren nach Anspruch 15, wobei das geeignete Gefäß zum Mischen der Inhaltsstoffe der Zusammensetzung das Druckreaktorgefäß ist.

18. Das Verfahren nach Anspruch 15, das weiter die Überführung des Inhaltes des Druckreaktorgefäßes in einen Behälter ohne signifikanten Verlust an dem Bicarbonat umfaßt.

19. Das Verfahren nach Anspruch 18, wobei der Behälter für das darin enthaltene Gas im wesentlichen undurchlässig ist.

20. Das Verfahren nach Anspruch 19, wobei der Behälter einen laminierten Folienbeutel umfaßt.

21. Eine Verpackung, die die ophthalmische Zusammensetzung nach einem der Ansprüche 1 bis 14 enthält, die im wesentlichen gegenüber Kohlendioxid undurchlässig ist, das oben enthalten und in der Zusammensetzung gelöst ist, so daß ein geschlossenes System geschaffen wird, in dem ein Gleichgewicht zwischen Kohlendioxid und Bicarbonat erreicht und aufrechterhalten werden kann, bis die Verpackung zum Gebrauch geöffnet wird.

## Revendications

1. Composition ophtalmique comprenant :
a) des ions potassium à une concentration d'environ 11 à environ 25 mmoles/l ;
b) des ions calcium à une concentration d'environ 0,2 à environ 0,5 mmole/l ;
c) des ions magnésium à une concentration d'environ 0,15 à environ 0,45 mmole/l ;
d) des ions bicarbonate à une concentration d'environ 1 à environ 36 mmoles/l ;
e) l'association : d'un polymère cellulosique et d'un glycosaminoglycane, ou d'un polymère cellulosique et d'un polymère carboxyvinylique ; et ayant
f) une viscosité d'environ 5 à environ 10 000 centipoises.

2. Composition suivant la revendication 1, dans laquelle la concentration d'ions bicarbonate est comrpise dans l'intervalle de 6 à 24 mmoles/l.

3. Composition suivant la revendication 1, comprenant en outre des ions zinc à une concentration d'environ 0,005 à environ 0,015 mmole/l.

4. Composition suivant la revendication 1, dans laquelle la concentration d'ions potassium est égale à environ 17,4 mmoles/l, la concentration d'ions calcium est égale à environ 0,36 mmole/l, la concentration d'ions magnésium est égale à environ 0,31 mmole/l et la concentration d'ions bicarbonate est égale à environ 11,9 mmoles/l.

5. Composition suivant la revendication 1, dans laquelle :
a) le rapport des concentrations molaires du potassium au bicarbonate est compris dans l'intervalle d'environ 1:0,04 à environ 1:3,27 ;
b) le rapport des concentrations molaires du calcium au magnésium est compris dans l'intervalle d'environ 1:0,3 à environ 1:2,25 ;
c) le rapport des concentrations molaires du potassium au calcium est compris dans l'intervalle d'environ 1:0,008 à environ 1:0,045 ; et
d) le rapport des concentrations molaires du bicarbonate au calcium est compris dans l'intervalle d'environ 1:0,0056 à environ 1:0,5.

6. Composition suivant la revendication 5, dans laquelle le rapport des concentrations molaires du potassium au bicarbonate est égal à environ 1:0,68, le rapport des concentrations molaires du calcium au magnésium est égal à environ 1:0,86, le rapport des concentrations molaires du potassium au calcium est égal à environ 1:0,02 et le rapport des concentrations molaires du bicarbonate au calcium est égal à environ 1:0,03.

7. Composition suivant la revendication 5, dans laquelle :
a) le rapport des concentrations molaires du potassium au bicarbonate est compris dans l'intervalle d'environ 1:0,24 à environ 1:2,18 ; et
b) le rapport des concentrations molaires du bicarbonate au calcium est compris dans l'intervalle d'environ 1:0,008 à environ 1:0,08.

8. Composition suivant la revendication 1, qui a une viscosité comprise dans l'intervalle d'environ 3000 à environ 6000 centipoises.

9. Composition suivant la revendication 1, dans laquelle le polymère cellulosique consiste en hydroxypropylméthylcellulose, hydroxyéthylcellulose, hydroxypropylcellulose ou méthylcellulose.

10. Composition suivant la revendication 1, dans laquelle le polymère carboxyvinylique consiste en carbomère 910, carbomère 940, carbomère 934P, carbomère 974P, carbomère 980 ou carbomère 1342.

11. Composition suivant la revendication 1, dans laquelle le polymère cellulosique comprend l'hydroxypropylméthylcellulose et le polymère carboxyvinylique comprend le carbomère 934P.

12. Composition suivant la revendication 1, dans laquelle le polymère cellulosique est présent à une concentration d'environ 0,5 % en poids.

13. Composition suivant la revendication 1, dans laquelle le polymère carboxyvinylique est présent à une concentration d'environ 0,175 % en poids.

14. Composition suivant la revendication 1, dans laquelle le glycosaminoglycane est présent dans la composition à une concentration comprise dans l'intervalle d'environ 0,1 à 5 % en poids.

15. Procédé de préparation d'une composition ophtalmique suivant l'une quelconque des revendications 1 à 14, dans lequel la composition est préparée par les étapes suivantes :
i) mélange des ingrédients de la composition dans un récipient convenable ;
ii) introduction de la composition dans une cuve de réacteur résistant à la pression ;
iii) introduction d'une quantité de gaz dans la cuve de réacteur résistant à la pression, la quantité du gaz étant suffisante pour induire un état d'équilibre désiré entre le gaz et le bicarbonate dans le système clos de la cuve de réacteur résistant à la pression ; et
iv) mélange du contenu de la cuve de réacteur résistant à la pression pendant un temps suffisant pour induire l'état d'équilibre entre le gaz et le bicarbonate.

16. Procédé suivant la revendication 15, dans lequel le gaz consiste en dioxyde de carbone.

17. Procédé suivant la revendication 15, dans lequel le récipient convenable pour le mélange des ingrédients de la composition est la cuve du réacteur résistant à la pression.

18. Procédé suivant la revendication 15, comprenant en outre le transfert du contenu de la cuve du réacteur résistant à la pression dans un réservoir sans perte notable du bicarbonate.

19. Procédé suivant la revendication 18, dans lequel le réservoir est pratiquement imperméable au gaz qu'il renferme.

20. Procédé suivant la revendication 19, dans lequel le réservoir comprend un sachet constitué d'une feuille métallique stratifiée.

21. Emballage comprenant la composition ophtalmique suivant l'une quelconque des revendications 1 à 14, pratiquement imperméable au dioxyde de carbone présent au-dessus de, et dissous dans, la composition, de telle sorte que soit engendré un système clos dans lequel un équilibre entre le dioxyde de carbone et le bicarbonate peut être atteint et maintenu jusqu'à l'ouverture de l'emballage pour son utilisation.
